# EUROPÄISCHE PATENTSCHRIFT

(11) **EP 2 794 547 B1**
(45) Veröffentlichungstag und Bekanntmachung des Hinweises auf die Patenterteilung: **06.02.2019**
(21) Anmeldenummer: 12801740.7
(22) Anmeldetag: 17.12.2012
(51) Int. Cl.: C07C 67/38, C07C 69/22, C07C 69/24, C07C 69/34, C07C 69/612, C07D 209/48

(54) **VERFAHREN ZUR HERSTELLUNG VON ESTERN AUS FORMIATEN UND OLEFINISCH UNGESÄTTIGTEN VERBINDUNGEN**
PROCESS FOR THE PREPARATION OF ESTERS FROM FORMATES AND COMPOUNDS OLEFINICALLY UNSATURATED
PROCÉDÉ DE PRÉPARATION D'ESTERS À PARTIR DE FORMATES ET DE COMPOSÉS OLÉFINES INSATURÉS.

(30) Priorität: 19.12.2011 DE 102011089008
(43) Veröffentlichungstag der Anmeldung: 29.10.2014
(73) Patentinhaber: Evonik Degussa GmbH, 45128 Essen (DE)
(72) Erfinder: FRANKE, Robert, 45772 Marl (DE); HESS, Dieter, 45770 Marl (DE); BELLER, Matthias, 18211 Nienhagen (DE); JACKSTELL, Ralf, 27478 Cuxhaven Altenwalde (DE); COZZULA, Daniela, 52062 Aachen (DE); FLEISCHER, Ivana, 93057 Regensburg (DE); JENNERJAHN, Reiko, 18190 Sanitz (DE)
(86) Internationale Anmeldenummer: PCT/EP2012/075766
(87) Internationale Veröffentlichungsnummer: WO 2013/092478

(56) Entgegenhaltungen:
- CHUL WOO LEE ET AL: "Hydroesterification of Olefins Catalyzed by Pd(OAc)2 Immobilized on Montmorillonite", THE JOURNAL OF ORGANIC CHEMISTRY, Bd. 60, Nr. 1, 1. Januar 1995 (1995-01-01) , Seiten 250-252, XP055054095, ISSN: 0022-3263, DOI: 10.1021/jo00106a042
- YUKO KATAFUCHI ET AL: "Palladium-Catalyzed Hydroesterification of Alkynes Employing Aryl Formates without the Use of External Carbon Monoxide", ADVANCED SYNTHESIS & CATALYSIS, Bd. 353, Nr. 2-3, 11. Februar 2011 (2011-02-11), Seiten 475-482, XP055053946, ISSN: 1615-4150, DOI: 10.1002/adsc.201000750
- IVAN J. B. LIN ET AL: "Regiochemical control in palladium(0) and palladium(II) catalysed alkene?formate ester carbonylation reactions", JOURNAL OF THE CHEMICAL SOCIETY, CHEMICAL COMMUNICATIONS, Nr. 4, 1. Januar 1989 (1989-01-01), Seite 248, XP055053947, ISSN: 0022-4936, DOI: 10.1039/c39890000248

## Beschreibung

Die vorliegende Erfindung betrifft ein Verfahren zur Herstellung von Estern aus Formiaten und olefinisch ungesättigten Verbindungen durch Carbonylierung mit Katalysatoren auf Basis von palladiumhaltigen Verbindungen.

### Stand der Technik

Ester, insbesondere solche die lineare Reste von vorzugsweise 8, 9 und 10 Kohlenstoffatomen tragen, haben technische Bedeutung als Weichmacher. Daneben werden lineare und verzweigte Ester für eine Vielzahl von Anwendungen als Spezial- und Feinchemikalien wie Arzneimittel, Duftstoffe und Insektiziden hergestellt. Die wichtigsten technisch angewandten Methoden zur Herstellung von linearen Estern sind die Veresterung von Carbonsäuren oder die direkte Methoxycarbonylierung von Olefinen in Gegenwart von Kohlenstoffmonoxid und einem Palladiumkatalysator; s. Abbildung 1. Einen Übersichtsartikel über die Methodik der Carbonylierung mit Formiaten findet man in Appl. Catal. A 1995 S. 25-44. Insgesamt werden pro Jahr mehr als 120.000 Tonnen Ester mittels Carbonylierung produziert.

In der Literatur gibt es sehr wenige Forschungsergebnisse in der Carbonylierung mit Formiaten. Katalysatoren, die in diesem Rahmen allgemein verwendet wurden, enthalten Ruthenium, Iridium oder Palladium in Gegenwart von verschiedensten Zusätzen, wie Promotern, Liganden oder Säuren. Alle bekannten Reaktionen setzen fast ausschließlich Ethylen als Olefinquelle um. Mit Zunahme der C-Atome im Olefin tritt bei allen Prozessen eine enorme Aktivitätsreduktion ein, begleitet von enormen Chemoselektivitätsverlusten. Da die meisten bekannten Systeme zur Carbonylierung mit Formiaten ligandfreie Systeme sind, ergeben sich hier neben schlechter Aktivität und Chemoselektivität bei höheren Olefinen auch noch schlechte Regioselektivitäten.

Die Herstellung von Estern aus Kohlenmonoxid und Methanol ist ein gut untersuchtes analoges Verfahren. Nachteilig sind jedoch die Notwendigkeit von teuren Hochdruck-Apparaturen, die Nutzung von reinem Kohlenmonoxid, welches ziemlich teuer, hochgiftig ist und aus fossilen Ressourcen hergestellt wird.

In CHUL WOO LEE ET AL, "Hydroesterification of Olefins Catalyzed by Pd(OAc)2 Immobilized on Montmorillonite", THE JOURNAL OF ORGANIC CHEMISTRY, (19950101), Bd. 60, Nr. 1, Seiten 250 - 252, wird ein Verfahren zur Herstellung von Estern aus Olefinen beschrieben. Hierbei kommen MeOH, CO und Pd(OAc)₂ zum Einsatz.

In YUKO KATAFUCHI ET AL, "Palladium-Catalyzed Hydroesterification of Alkynes Employing Aryl Formates without the Use of External Carbon Monoxide", ADVANCED SYNTHESIS & CATALYSIS, (20110211), Bd. 353, Nr. 2-3, Seiten 475 - 482, wird ein Verfahren zur Herstellung von Estern aus Alkinen beschrieben. Als CO-Quelle dienen hierbei Aryl-Fromiate.

In IVAN J. B. LIN ET AL, "Regiochemical control in palladium(0) and palladium(II) catalysed alkeneformate ester carbonylation reactions", JOURNAL OF THE CHEMICAL SOCIETY, CHEMICAL COMMUNICATIONS, (19890101), Nr. 4, Seiten 248 - 449, wird ein Verfahren zur Herstellung von Estern aus Olefinen beschrieben. Hierbei kommen Pd(PPh₃)₄ oder Pd(dba)₂ zum Einsatz.

Zusammenfassend kann festgestellt werden, dass kein Verfahren zur Hydroesterifizierung bekannt ist, welches auch höhere Olefine als Ethylen, insbesondere olefinhaltige Gemische, mit guten Chemoselektivitäten (>95%), guten Regioselektivitäten (>90%), guten Aktivitäten (TOF>100h⁻¹) und bei der Verwendung von olefinhaltigen Gemischen, welche innenständige Kohlenstoff-Kohlenstoff-Doppelbindungen aufweisen, unter isomerisierenden Bedingungen umsetzen kann, wobei kein Kohlenmonoxid als Edukt verwendet wird. Das Erreichen dieser Kennzahlen ist nötig, um industrielle Umsetzungen zu erreichen.

### Ziel der Erfindung

Aus den oben genannten Gründen besteht ein großer Bedarf nach neuen verbesserten Verfahren zur Carbonylierung von olefinisch ungesättigten Verbindungen, insbesondere olefinhaltigen Gemischen, die auch innenständige Kohlenstoff-Kohlenstoff-Doppelbindungen aufweisen, mit Formiaten. Ein besonderes Ziel liegt darin, auch langkettige Olefine mit mehr als 2 C-Atomen der Carbonylierung mit Formiaten, d.h. keine Verwendung von Kohlenmonoxid als Edukt, zugänglich zu machen. Zu berücksichtigen ist hierbei ebenfalls das Erreichen hoher n-Selektivitäten, d.h. die Bildung von n-terminalen Estern auch aus olefinhaltigen Gemischen, und entsprechenden Aktivitäten, die für eine technische Anwendung erforderlich sind.

### Zusammenfassung der Erfindung

Im Gegensatz zur gut untersuchten Herstellung von Estern aus Kohlenmonoxid und Methanol wird in dem hier präsentierten Verfahren lediglich ein Substrat benötigt, welches sich im flüssigen Aggregatszustand befindet. Dieses Substrat, das Formiat, ist ein Addukt aus Kohlenmonoxid und Alkohol. Es kann aus der Hydrierung von CO₂ gewonnen werden und stellt somit einen chemischen Prozess dar, der hilft Klimagase zu reduzieren. Die Herstellung von Estern aus Kohlenmonoxid und Methanol hingegen bezieht ihren Rohstoff Kohlenmonoxid hauptsächlich aus fossilen Ressourcen, wie der Kohlevergasung.

### Detaillierte Beschreibung der Erfindung

Die Erfindung betrifft ein Verfahren zur Herstellung von Estern durch Carbonylierung, dadurch gekennzeichnet, dass es
i) unter Verwendung mindestens einer palladiumhaltigen Verbindung,
ii) mindestens einer olefinisch ungesättigten Verbindung,
iii) mindestens eines phosphorhaltigen Liganden,
iv) mindestens eines Formiates,
v) mindestens eines Alkohols,
vi) mindestens einer Säure,
vii) in einem Temperaturbereich von 80° bis 120°C,
viii) bei einem Reaktionsdruck von 0,1 bis 0,6 MPa, durchgeführt wird,
wobei die Säure ausgewählt ist aus der Gruppe der Sulfonsäuren.

Der palladiumhaltige Katalysator umfaßt einen Phosphor enthaltenden Liganden und eine Säure im Verhältnis Palladium : Ligand im Bereich von 1 : 1,1 bis 1 : 100 und Palladium : Säure im Bereich von 1 : 1bis 1 : 1000 und alle Verhältnisse sind Molverhältnisse.
Die Zielreaktion läuft vorzugsweise bei Temperaturen von 60 bis 180°C ab; besonders bevorzugt bei 80 bis 120°C.

Für das erfindungsgemäße Verfahren können auch Lösungsmittel für den Katalysator eingesetzt werden. Als Lösungsmittel werden im Allgemeinen polare inerte organische Lösungsmittel, Wasser oder der dem jeweiligen Formiat entsprechende Alkohol, wie z.B. Methanol bei Methylformiat, Ethanol bei Ethylformiat, verwendet. Beispielhaft genannt werden dipolar aprotische Lösungsmittel, aliphatische Ether, Amide, aromatische Verbindungen, Alkohole und Ester sowie deren Gemische. Besonders bevorzugt sind die dem jeweiligen Formiat entsprechende Alkohole

Als Palladiumquelle können alle Palladium-enthaltende Salze und Komplexe als Vorstufe verwendet werden, die unter den Reaktionsbedingungen Palladiumhydrid-Komplexe bilden. Beispielhaft genannt werden Pd(II)-Halogenide (z. B. Pd(II)Cl₂), Pd(II)-Komplexe (z. B. Pd(II)acetylacetonat, Pd(II)acetat, Pd(II)-Dibenzylidenaceton), Pd(0)- Komplexe (z.B. Pd(0)[PPh₃]₄). Die Palladiumverbindungen können in unterschiedlichen Oxidationsstufen von 0 bis +II vorliegen, die mit der Säure und dem Formiat zu den entsprechenden aktiven Palladium-Hydrid-Komplex reagieren.

Eine besonders bevorzugte Vorstufe ist Palladium-acetylacetonat.

Um die gewünschten Katalysatorselektivitäten und Katalysatoraktivitäten zu erreichen, ist es notwendig einen Phosphor-enthaltenden Liganden zuzusetzen. Im vorliegenden Verfahren wird dieser Ligand im Überschuß zum Palladium eingesetzt. Das Verhältnis Palladium zu Ligand beträgt vorzugsweise zwischen 1:2 bis 1:50.

Als Liganden können beliebige, dreiwertige Phosphor enthaltende Liganden eingesetzt werden, die eine koordinative Bindung zum Palladiumzentrum ausbilden können. Beispielhaft sei hier genannt α,α'-bis(di-t-butylphosphino)-o-Xylol, dargestellt durch Formel **1**, später mit BuPoX abgekürzt. Die Liganden können dabei sowohl monodentat als auch multidentat binden. Bevorzugt sind bidentate Liganden.

Ein besonders bevorzugter Ligand ist α,α'-bis(di-t-butylphosphino)-o-Xylol, dargestellt durch Formel **1.** 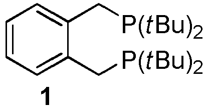 Insbesondere bevorzugt sind aus der Gruppe der Sulfonsäuren Methansulfonsäure, p-Toluolsulfonsäure.

Ungesättigte Verbindungen, die mit den genannten Katalysatorsystemen selektiv umgesetzt werden können sind die Klasse der Olefine. Besonders bevorzugt sind terminale Alkene und aromatische Olefine mit einer Kohlenstoffanzahl zwischen 2 und 20 und deren Gemische. Besonders bevorzugt sind Olefine mit 6 bis 12 Kohlenstoffatomen sowie deren Gemische. Verzweigte und interne Olefine können ebenfalls umgesetzt werden.

Das erfindungsgemäße Verfahren hat sich besonders für die Herstellung von Estern mit 3 bis 21 Kohlenstoffatomen bewährt. Insbesondere ist die Herstellung von Estern mit 7 bis 13 Kohlenstoffatomen bevorzugt.

Die Olefine können funktionalisiert sein und neben Kohlenstoff sowie Wasserstoff weitere Heteroatome, wie z.B. Stickstoff und/oder Sauerstoff aufweisen. Ohne Anspruch auf Vollständigkeit seien hier als Substrate ungesättigte Alkohole, Ether, Amine, Ester, Carbonsäuren, Amide, Urethane, Halogenide, Aldehyde, Ketone und Epoxide genannt.

Bei dem erfindungsgemäßen Verfahren können im batch-Versuch Turnover-Werte [(TON) = Produkt (mol) / Palladium (mol)] der Katalysatoren in der Größenordnung von 3.400 und mehr realisiert werden. Daher werden typischerweise zwischen 0,038 mol% Palladium (bezogen auf Olefinsubstrat) eingesetzt.

Aufgrund der guten Katalysatoraktivitäten ist es bei dem erfindungsgemäßen Verfahren möglich, sehr kleine Mengen an Katalysator zu verwenden.

Das erfindungsgemäße Verfahren ist insofern besonders überraschend und neu, da in der Vergangenheit keine längerkettigen und hoch stereo- und regioselektiven Ester von Olefinen mit ausreichender Aktivität beschrieben wurden. Das beschriebene Verfahren zeigt hier erstmals, daß unter den erfindungsgemäßen Bedingungen gute Ausbeuten und n-Selektivitäten an n-terminalen Estern möglich sind. Die besonderen Vorteile des neuen Verfahrens bestehen darin, daß keine Gase, insbesondere Kohlenmonoxid, als Edukt mehr benötigt werden, um eine Carbonylierung durchzuführen.

Zusätzlich ist es möglich die Formiate aus dem Klimagas CO₂ herzustellen. Somit erhält man die Möglichkeit bei der Herstellung von Estern einen umweltfreundlichen und weniger komplexen Prozeß zu betreiben.

Für eine technische Anwendung ist es ebenfalls notwendig, daß hohe Katalysatoraktivitäten erzielt werden. Dies gelingt durch das erfindungsgemäße Verfahren. So wird beispielsweise 1-Octen mit einer Katalysatorfrequenz bzw. Reaktionsgeschwindigkeitskonstante von über 209h⁻¹ und einer Umsatzzahl im Batchversuch von 3400. Es ergab sich somit eine Raumzeitausbeute von 16.2 g/(l*h) bzw.0.016 t/(m³*h).

Die erfindungsgemäß hergestellten Ester können unter anderem als Zwischenprodukte für Weichmacheralkohole eingesetzt werden und als Vorprodukte für Pharmazeutika und Agrochemikalien sowie Bausteine für Polymere.

### Beispiele:

Die nachstehenden Beispiele dienen zur Erläuterung des erfindungsgemäßen Verfahrens.

Allgemeine Arbeitsvorschrift zur Herstellung von Estern aus Formiat und olefinisch ungesättigten Verbindungen mittels eines Palladium/Phosphin/Säure-Katalysators:
Es werden 54.5 mmol 1-Octen (8,5mL), Pd(acac)₂ 0,038 mol% (6,3 mg), 0,13 mol% 1 = BuPoX (28,4 mg), 10 ml Methylformiat, 10ml Methanol und 20µl Methansulfonsäure unter Schutzgas (z. B. Argon oder Stickstoff) in einen 100 ml Edelstahlautoklav überführt. Der Autoklav wird auf 100 °C erwärmt, wobei ein Enddruck von 0,51 MPa erreicht und bei dieser Temperatur 20 h gerührt wird. Anschließend wird der Autoklav auf Raumtemperatur abgekühlt und der Restdruck abgelassen. Es werden 5 ml Isooctan zur Reaktionslösung als interner Standard zugesetzt und das Gemisch wird gaschromatografisch untersucht.

Allgemeine Angaben siehe Anmerkung [a] der Tabelle 1.

In der nachfolgenden Tabelle 1 werden Änderungen einiger Reaktionsparameter, wie z.B. die Variation von Liganden der folgenden Strukturen dargestellt:

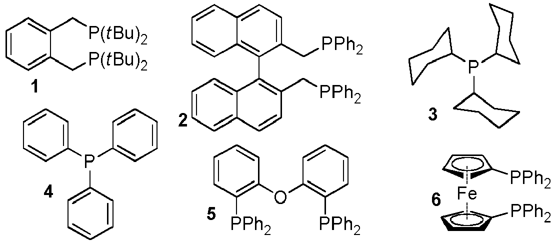

Das mögliche Produktspektrum des erfindungsgemäßen Verfahrens bei Verwendung von 1-Octen und Methylformiat ergibt sich gemäß der Reaktion **(1)**:

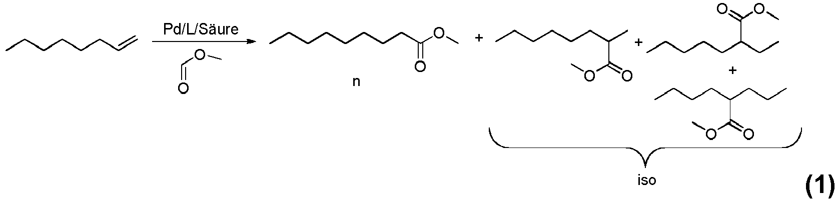

**Tabelle 1 Pd-katalysierte Methoxycarbonylierung von 1-Octen.**

| Beispiel | Ligand | Säure | T [°C] | MeOH/MF [mL/mL] | Ausb eute [%]^{[b]} | n-Sel. [%] |
|---|---|---|---|---|---|---|
| 1 | 1 | Me-SO₃H | 100 | 10/10 | 46^{[c]} | 95 |
| 2 | 1 | pTsO H | 100 | 10/10 | 36^{[c]} | 95 |
| 3 | 1 | HOAc | 100 | 10/10 | 0 | - |
| 4 | 1 | - | 100 | 10/10 | 0 | - |
| 5 | 1 | Me-SO₃H | 80 | 10/10 | 34 | 95 |
| 6 | 1 | Me-SO₃H | 100 | 10/10 | 98 | 94 |
| 7 | 1 | Me-SO₃H | 120 | 10/10 | 79 | 93 |
| 8 | 1 | Me-SO₃H | 100 | 0/10 | 28 | 95 |
| 9 | 1 | Me-SO₃H | 100 | 10/10 | 98 | 94 |
| 10 | 2 | Me-SO₃H | 100 | 10/10 | 0 | - |
| 11 | 3 | Me-SO₃H | 100 | 10/10 | 0 | - |
| 12 | 4 | Me-SO₃H | 100 | 10/10 | 0 | - |
| 13 | 5 | Me-SO₃H | 100 | 10/10 | 0 | - |
| 14 | 6 | Me-SO₃H | 100 | 10/10 | 0 | - |

| | | | | | | |
|---|---|---|---|---|---|---|
| [a] soweit nicht anders beschrieben wurden die Reaktionen bei 100°C mit 0.038 mol%, Pd(acac)₂, L/Pd = 4 (L = BuPoX = **1**),10 mL Methylformiat, 10mL Methanol, Säure/L = 4 (= 20 µl MeSO₃H), 54 mmol Olefin durchgeführt. [b] mit gaschromatografischer Methode unter Verwendung eines internen Standards bestimmt. [c] Ausbeuten nach einer Reaktionszeit von 5.5h. | | | | | | |

*Beispiel 1:* Es werden 54.5 mmol 1-Octen(8,5mL), Pd(acac)₂ 0,038 mol% (6,3 mg), 0,13 mol% BuPoX (28,4 mg), 10 mL Methylformiat, 10mL Methanol und 20µL Methansulfonsäure unter Schutzgas (z. B. Argon oder Stickstoff) in einen 100 mL Edelstahlautoklav überführt. Der Autoklav wird auf 100 °C erwärmt, wobeiein Enddruck von 5,1bar erreicht,und bei dieser Temperatur 5.5 h gerührt wird. Anschließend wird der Autoklav abgekühlt auf Raumtemperatur und der Restdruck abgelassen. Es werden 5 mL Isooctan zur Reaktionslösung als interner Standard zugesetzt und das Gemisch wird gaschromatografisch untersucht. Die Ausbeute an n-Produkt, dem Nonansäuremethylester beträgt 43,7 %. Die Ausbeute der verzweigten Produkte (2-Methyl-octansäure-methylester, 2-Ethyl-heptansäure-methylester und 2-Propyl-hexansäure-methylester) beträgt zusammen 2,3%. Somit Ergibt sich eine Gesamtausbeute an Methylestern von 46% mit einem n: iso Verhältnis von 95 : 5.
*Beispiel 2:* Es werden 54.5 mmol 1-Octen(8,5mL), Pd(acac)₂ 0,038 mol% (6,3 mg), 0,13 mol% BuPoX (28,4 mg), 10 mL Methylformiat, 10mL Methanol und 58mg p-Toluolsulfonsäure unter Schutzgas (z. B. Argon oder Stickstoff) in einen 100 mL Edelstahlautoklav überführt. Der Autoklav wird auf 100 °C erwärmt, wobei ein Enddruck von 5,1bar erreicht und bei dieser Temperatur 5.5 h gerührt wird. Anschließend wird der Autoklav abgekühlt auf Raumtemperatur und der Restdruck abgelassen. Es werden 5 mL Isooctan zur Reaktionslösung als interner Standard zugesetzt und das Gemisch wird gaschromatografisch untersucht. Die Ausbeute der verzweigten Produkte (2-Methyl-octansäure-methylester, 2-Ethyl-heptansäure-methylester und 2-Propyl-hexansäure-methylester) beträgt zusammen 1,8%. Somit Ergibt sich eine Gesamtausbeute an Methylestern von 36% mit einem n: iso Verhältnis von 95 : 5.
*Beispiel 3 (Vergleichsbeispiel):* Es werden 54.5 mmol 1-Octen(8,5mL), Pd(acac)₂ 0,038 mol% (6,3 mg), 0,13 mol% BuPoX (28,4 mg), 10 mL Methylformiat, 10mL Methanol und 17.6 µL Essigsäure unter Schutzgas (z. B. Argon oder Stickstoff) in einen 100 mL Edelstahlautoklav überführt. Der Autoklav wird auf 100 °C erwärmt, wobei ein Enddruck von 5,1bar erreicht und bei dieser Temperatur 5.5 h gerührt wird. Anschließend wird der Autoklav abgekühlt auf Raumtemperatur und der Restdruck abgelassen. Es werden 5 mL Isooctan zur Reaktionslösung als interner Standard zugesetzt und das Gemisch wird gaschromatografisch untersucht. Die Ausbeute an Nonansäuremethylester beträgt 0 %.
*Beispiel 4 (Vergleichsbeispiel):* Es werden 54.5 mmol 1-Octen(8,5mL), Pd(acac)₂ 0,038 mol% (6,3 mg), 0,13 mol% BuPoX (28,4 mg), 10 mL Methylformiat, 10mL Methanol und ohne Säure unter Schutzgas (z. B. Argon oder Stickstoff) in einen 100 mL Edelstahlautoklav überführt. Der Autoklav wird auf 100 °C erwärmt, wobei ein Enddruck von 5,1bar erreicht und bei dieser Temperatur 20 h gerührt wird. Anschließend wird der Autoklav abgekühlt auf Raumtemperatur und der Restdruck abgelassen. Es werden 5 mL Isooctan zur Reaktionslösung als interner Standard zugesetzt und das Gemisch wird gaschromatografisch untersucht. Die Ausbeute an Nonansäuremethylester beträgt 0 %.
*Beispiel 5:* Es werden 54.5 mmol 1-Octen(8,5mL), Pd(acac)₂ 0,038 mol% (6,3 mg), 0,13 mol% BuPoX (28,4 mg), 10 mL Methylformiat, 10mL Methanol und 20µL Methansulfonsäure unter Schutzgas (z. B. Argon oder Stickstoff) in einen 100 mL Edelstahlautoklav überführt. Der Autoklav wird auf 80 °C erwärmt und bei dieser Temperatur 20 h gerührt. Anschließend wird der Autoklav abgekühlt auf Raumtemperatur und der Restdruck abgelassen. Es werden 5 mL Isooctan zur Reaktionslösung als interner Standard zugesetzt und das Gemisch wird gaschromatografisch untersucht. Die Ausbeute an n-Produkt, dem Nonansäuremethylester beträgt 32,3 %. Die Ausbeute der verzweigten Produkte (2-Methyl-octansäure-methylester, 2-Ethyl-heptansäure-methylester und 2-Propyl-hexansäure-methylester) beträgt zusammen 1,7%. Somit Ergibt sich eine Gesamtausbeute an Methylestern von 34% mit einem n: iso Verhältnis von 95 : 5.
*Beispiel 6:* Es werden 54.5 mmol 1-Octen(8,5mL), Pd(acac)₂ 0,038 mol% (6,3 mg), 0,13 mol% BuPoX (28,4 mg), 10 mL Methylformiat, 10mL Methanol und 20µL Methansulfonsäure unter Schutzgas (z. B. Argon oder Stickstoff) in einen 100 mL Edelstahlautoklav überführt. Der Autoklav wird auf 100 °C erwärmt, wobei ein Enddruck von 5,1bar erreicht und bei dieser Temperatur 20 h gerührt wird. Anschließend wird der Autoklav abgekühlt auf Raumtemperatur und der Restdruck abgelassen. Es werden 5 mL Isooctan zur Reaktionslösung als interner Standard zugesetzt und das Gemisch wird gaschromatografisch untersucht. Die Ausbeute an n-Produkt, dem Nonansäuremethylester beträgt 92,1 %. Die Ausbeute der verzweigten Produkte (2-Methyl-octansäure-methylester, 2-Ethyl-heptansäure-methylester und 2-Propyl-hexansäure-methylester) beträgt zusammen 5,9 %. Somit Ergibt sich eine Gesamtausbeute an Methylestern von 98% mit einem n: iso Verhältnis von 94 : 6.
*Beispiel* 7: Es werden 54.5 mmol 1-Octen(8,5mL), Pd(acac)₂ 0,038 mol% (6,3 mg), 0,13 mol% BuPoX (28,4 mg), 10 mL Methylformiat, 10mL Methanol und 20µL Methansulfonsäure unter Schutzgas (z. B. Argon oder Stickstoff) in einen 100 mL Edelstahlautoklav überführt. Der Autoklav wird auf 120 °C erwärmt, wobei ein Enddruck von 5,1bar erreicht und bei dieser Temperatur 20 h gerührt wird. Anschließend wird der Autoklav abgekühlt auf Raumtemperatur und der Restdruck abgelassen. Es werden 5 mL Isooctan zur Reaktionslösung als interner Standard zugesetzt und das Gemisch wird gaschromatografisch untersucht. Die Ausbeute an n-Produkt, dem Nonansäuremethylester beträgt 73,5 %. Die Ausbeute der verzweigten Produkte (2-Methyl-octansäure-methylester, 2-Ethyl-heptansäure-methylester und 2-Propyl-hexansäure-methylester) beträgt zusammen 5,5 %. Somit Ergibt sich eine Gesamtausbeute an Methylestern von 79 % mit einem n: iso Verhältnis von 93 : 7.
*Beispiel 8:* Es werden 54.5 mmol 1-Octen(8,5mL), Pd(acac)₂ 0,038 mol% (6,3 mg), 0,13 mol% BuPoX (28,4 mg), 10 mL Methylformiat und 20µL Methansulfonsäure unter Schutzgas (z. B. Argon oder Stickstoff) in einen 100 mL Edelstahlautoklav überführt. Der Autoklav wird auf 100 °C erwärmt und bei dieser Temperatur 20 h gerührt. Anschließend wird der Autoklav abgekühlt auf Raumtemperatur und der Restdruck abgelassen. Es werden 5 mL Isooctan zur Reaktionslösung als interner Standard zugesetzt und das Gemisch wird gaschromatografisch untersucht. Die Ausbeute an Nonansäuremethylester beträgt 28 % mit einem n: iso Verhältnis von 95 : 5.
*Beispiel 9:* entspricht Beispiel 6
*Beispiel 10-14:* Es werden 54.5 mmol 1-Octen(8,5mL), Pd(acac)₂ 0,038 mol% (6,3 mg), Ligand (Beispiel 10: 55 mg Ligand **2**; Beispiel 11: 116 mg Ligand **3**; Beispiel 12: 163 mg Ligand **4**; Beispiel 13: 44.6 mg Ligand **5**; Beispiel 14: 39.2mg Ligand **6**), 10 mL Methylformiat, 10mL Methanol und 20µL Methansulfonsäure unter Schutzgas (z. B. Argon oder Stickstoff) in einen 100 mL Edelstahlautoklav überführt. Der Autoklav wird auf 100 °C erwärmt, wobei ein Enddruck von 5,1bar erreicht und bei dieser Temperatur 20 h gerührt wird. Anschließend wird der Autoklav abgekühlt auf Raumtemperatur und der Restdruck abgelassen. Es werden 5 mL Isooctan zur Reaktionslösung als interner Standard zugesetzt und das Gemisch wird gaschromatografisch untersucht. Die Ausbeute an Nonansäuremethylester beträgt in allen Fällen 0%.

Da in der Industrie häufig Olefingemische Verwendung finden, ist eine Kernkompetenz eines industriell nutzbaren Katalysators die gute Isomerisierung von Olefinen unter gleichzeitiger hochselektiver n-terminaler Funktionalisierung.

In einer besonderen Ausführungsform der Erfindung wird ein olefinhaltiges Gemisch, welches innenständige Kohlenstoff-Kohlenstoff-Doppelbindungen aufweist, als olefinisch ungesättigte Verbindung verwendet. Die Leistungsfähigkeit des vorgestellten Systems wird in Abbildung 2 an einer solchen technischen Mischung demonstriert; s.a. Beispiel 16.

Die Reaktion **(2)** gibt den allgemeinen Verlauf an.

Die Substituenten R₁, R₂ und R₃ entsprechen den Gruppen oder Teilen der Verbindungen, die in den nachfolgenden Beispielen 15 bis 23 aus den Spalten "Olefin" und "Produkt" zu entnehmen sind.

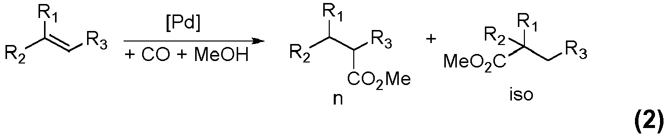

In den Beispielen der Tabelle 2 sind die ungesättigten Ausgangsverbindungen (Olefin und Formiat) und die erhaltenen Produkte aufgeführt. Die Spalte n-Selektivität gibt die Anteile Produkt mit n-terminaler Estergruppe an.

**Tabelle 2**

| **Beispiel** | **Olefin** | **Formates ter** | **Ausb eute [%]^{[b]}** | **n-Selektivität** | **Produkt** |
|---|---|---|---|---|---|
| **15** | 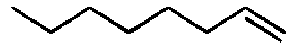 | 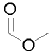 | **98** | **94** | 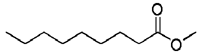 |
| **16** | 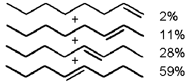 | 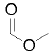 | **80** | **94** | 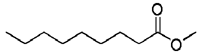 |
| **17** | 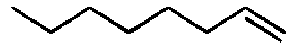 | 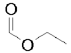 | **99** | **95** | 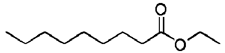 |
| **18** | 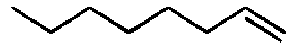 | 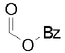 | **86** | **92** | 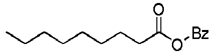 |
| **19** | 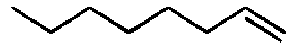 | 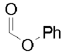 | **46** | **93** | 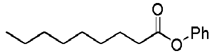 |
| **20** | 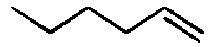 | 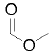 | **86** | **95** | 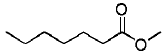 |
| **21^{[e]}** | 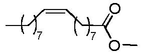 | 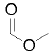 | **82** | **88** | 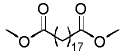 |
| **22^{[c]}** | 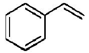 | 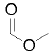 | **98** | **89** | 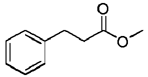 |
| **23^{[c]}** | 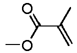 | 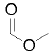 | **81** | **>99** | 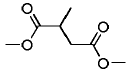 |
| **24^{[c]}** | 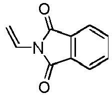 | 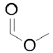 | **56** | **>99** | 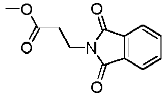 |

| | | | | | |
|---|---|---|---|---|---|
| [a] Reaktionen wurden bei 100°C mit 6.3 mg (0.038 mol% bei Verwendung von 54mmol Olefin) Pd(acac)₂, L/Pd = 4 (L = **1** = BuPoX),10 mL Formiat, 10mL Alkohol (entsprechender Alkohol zum verwendeten Formiat), Säure/L = 4 (= 20 µl MeSO₃H), 54 mmol Olefin. [b] mit gaschromatografischer Methode unter Verwendung eines internen Standards bestimmt. [c] 27 mmol olefin [d] Bedingungen Formiatscreening [e] 10mmol Methyloleate, 0.3 mol% Pd(acac)₂ Reaktionszeit 166h. | | | | | |

*Beispiel 15:* entspricht Beispiel 6
*Beispiel 16:* Es werden 54.5 mmol Octen-gemisch (8,5mL bestehend aus: 2% 1-Octen 11% 2-Octen 28% 3-Octen 59% 4-Octen), Pd(acac)₂ 0,038 mol% (6,3 mg), 0,13 mol% BuPoX (28,4 mg), 10 ml Methylformiat, 10ml Methanol und 20µl Methansulfonsäure unter Schutzgas (z. B. Argon oder Stickstoff) in einen 100 ml Edelstahlautoklav überführt. Der Autoklav wird auf 100 °C erwärmt, wobei ein Enddruck von 5,1bar erreicht und bei dieser Temperatur 20 h gerührt wird. Anschließend wird der Autoklav abgekühlt auf Raumtemperatur und der Restdruck abgelassen. Es werden 5 ml Isooctan zur Reaktionslösung als interner Standard zugesetzt und das Gemisch wird gaschromatografisch untersucht. Die Ausbeute an n-Produkt, dem Nonansäuremethylester beträgt 75,2 %. Die Ausbeute der verzweigten Produkte (2-Methyl-octansäure-methylester, 2-Ethyl-heptansäure-methylester und 2-Propyl-hexansäure-methylester) beträgt zusammen 4,8 %. Somit ergibt sich eine Gesamtausbeute an Methylestern von 80% mit einem n: iso Verhältnis von 94 : 6.
*Beispiel 17*: Es werden 19.3 mmol 1-Octen(3mL), Pd(acac)₂ 0,16 mol% (9,4 mg), 124µmol BuPoX (49 mg), 10 mL Ethylformiat, 10mL Ethanol und 28µL Methansulfonsäure unter Schutzgas (z. B. Argon oder Stickstoff) in einen 100 mL Edelstahlautoklav überführt. Der Autoklav wird auf 120 °C erwärmt und bei dieser Temperatur 20 h gerührt. Anschließend wird der Autoklav abgekühlt auf Raumtemperatur und der Restdruck abgelassen. Es werden 5 mL Isooctan zur Reaktionslösung als interner Standard zugesetzt und das Gemisch wird gaschromatografisch untersucht. Die Ausbeute an n-Produkt, dem Nonansäureethylester beträgt 94,1 %. Die Ausbeute der verzweigten Produkte (2-Methyl-octansäure-ethylester, 2-Ethyl-heptansäure-ethylester und 2-Propyl-hexansäure-ethylester) beträgt zusammen 4,9 %. Somit Ergibt sich eine Gesamtausbeute an Ethylestern von 99 % mit einem n: iso Verhältnis von 95 : 5.
*Beispiel 18*: Es werden 19.3 mmol 1-Octen(3mL), Pd(acac)₂ 0,16 mol% (9,4 mg), 124µmol BuPoX (49 mg), 10 mL Benzylformiat, 10mL Benzylakohol und 28µL Methansulfonsäure unter Schutzgas (z. B. Argon oder Stickstoff) in einen 100 mL Edelstahlautoklav überführt. Der Autoklav wird auf 120 °C erwärmt und bei dieser Temperatur 20 h gerührt. Anschließend wird der Autoklav abgekühlt auf Raumtemperatur und der Restdruck abgelassen. Es werden 5 mL Isooctan zur Reaktionslösung als interner Standard zugesetzt und das Gemisch wird gaschromatografisch untersucht. Die Ausbeute der verzweigten Produkte (2-Methyl-octansäure-benzylester, 2-Ethyl-heptansäurebenzylester und 2-Propyl-hexansäure-benzylester) beträgt zusammen 6,9 %. Somit Ergibt sich eine Gesamtausbeute an Benzylestern von 86 % mit einem n: iso Verhältnis von 92 : 8.
*Beispiel 19*: Es werden 19.3 mmol 1-Octen(3mL), Pd(acac)₂ 0,16 mol% (9,4 mg), 124µmol BuPoX (49 mg), 10 mL Phenylformiat, 10mL Phenol und 28µL Methansulfonsäure unter Schutzgas (z. B. Argon oder Stickstoff) in einen 100 mL Edelstahlautoklav überführt. Der Autoklav wird auf 90 °C und bei dieser Temperatur 20 h gerührt. Anschließend wird der Autoklav abgekühlt auf Raumtemperatur und der Restdruck abgelassen. Es werden 5 mL Isooctan zur Reaktionslösung als interner Standard zugesetzt und das Gemisch wird gaschromatografisch untersucht. Die Ausbeute an n-Produkt, dem Nonansäurephenylester beträgt 42,8 %. Die Ausbeute der verzweigten Produkte (2-Methyl-octansäure-phenylester, 2-Ethyl-heptansäure-phenylester und 2-Propyl-hexansäure-phenylester) beträgt zusammen 3,2 %. Somit ergibt sich eine Gesamtausbeute an Phenylestern von 46 % mit einem n: iso Verhältnis von 93 : 7.
*Beispiel 20:* Es werden 54.8 mmol 1-Hexen(6.8mL), Pd(acac)₂ 0,038 mol% (6,3 mg), 0,13 mol% BuPoX (28,4 mg), 10 mL Methylformiat, 10mL Methanol und 20µL Methansulfonsäure unter Schutzgas (z. B. Argon oder Stickstoff) in einen 100 mL Edelstahlautoklav überführt. Der Autoklav wird auf 100 °C erwärmt und bei dieser Temperatur 20 h gerührt. Anschließend wird der Autoklav abgekühlt auf Raumtemperatur und der Restdruck abgelassen. Es werden 5 mL Isooctan zur Reaktionslösung als interner Standard zugesetzt und das Gemisch wird gaschromatografisch untersucht. Die Ausbeute an n-Produkt, dem Heptansäuremethylester beträgt 81,7 %. Die Ausbeute der verzweigten Produkte (2-Methyl-hexansäure-methylester und 2-Ethyl-pentansäure-methylester) beträgt zusammen 4,3 %. Somit ergibt sich eine Gesamtausbeute an Methylestern von 86 % mit einem n: iso Verhältnis von 95 : 5.
*Beispiel 21:* Es werden 10 mmol Methyloleat(3.4mL), 9,4 mg Pd(acac)₂, 124µmol BuPoX (49 mg) 10 mL Methylformiat, 10mL Methanol und 26µL Methansulfonsäure unter Schutzgas (z. B. Argon oder Stickstoff) in einen 100 mL Edelstahlautoklav überführt. Der Autoklav wird auf 100 °C erwärmt und bei dieser Temperatur 166 h gerührt. Anschließend wird der Autoklav abgekühlt auf Raumtemperatur und der Restdruck abgelassen. Das Produkt ist als Feststoff ausgefallen. Es werden 5 mL Isooctan zur Reaktionslösung als interner Standard zugesetzt und soviel Methanol zugefügt, bis alles gelöst ist. Das Gemisch wird nun gaschromatografisch untersucht. Die Ausbeute an n-Produkt, dem Eicosandisäure-dimethylester beträgt 72,2 %. Die Ausbeute der verzweigten Produkte (beispielhaft 2-Methyl-nonadecandisäure-dimethylester, 2-Ethyloctadecandisäure-dimethylester) beträgt zusammen 9,8 %. Somit ergibt sich eine Gesamtausbeute an Methylestern von 82 % mit einem n: iso Verhältnis von 88 : 12.
*Beispiel 22*: Es werden 27 mmol Styrol(3.1 mL), Pd(acac)₂ 0,08 mol% (6,3 mg), 28,4 mg BuPoX, 10 mL Methylformiat, 10mL Methanol und 20µL Methansulfonsäure unter Schutzgas (z. B. Argon oder Stickstoff) in einen 100 mL Edelstahlautoklav überführt. Der Autoklav wird auf 100 °C erwärmt und bei dieser Temperatur 20 h gerührt. Anschließend wird der Autoklav abgekühlt auf Raumtemperatur und der Restdruck abgelassen. Es werden 5 mL Isooctan zur Reaktionslösung als interner Standard zugesetzt und das Gemisch wird gaschromatografisch untersucht. Die Ausbeute an n-Produkt, dem 3-Phenyl-propionsäuremethylester beträgt 87,2 %. Die Ausbeute des verzweigten Produktes 2-Phenyl-propionsäuremethylester beträgt 10,8 %. Somit ergibt sich eine Gesamtausbeute an Methylestern von 98 % mit einem n: iso Verhältnis von 89 : 11.
*Beispiel 23:* Es werden 27.2 mmol Methyl-methacrylat(2.9mL), Pd(acac)₂ 0,08 mol% (6,3 mg), 28,4 mg BuPoX, 10 mL Methylformiat, 10mL Methanol und 20µL Methansulfonsäure unter Schutzgas (z. B. Argon oder Stickstoff) in einen 100 mL Edelstahlautoklav überführt. Der Autoklav wird auf 100 °C erwärmt und bei dieser Temperatur 20 h gerührt. Anschließend wird der Autoklav abgekühlt auf Raumtemperatur und der Restdruck abgelassen. Es werden 5 mL Isooctan zur Reaktionslösung als interner Standard zugesetzt und das Gemisch wird gaschromatografisch untersucht. Die Ausbeute an n-Produkt, dem 2-methyl-succinsäuredimethylester beträgt 81 %. Verzweigte Produkte waren nicht festzustellen. Somit ergibt sich eine Gesamtausbeute an Methylestern von 81 % mit einem n: iso Verhältnis von 100 : 0.
*Beispiel 24*: Es werden 27 mmol N-Vinylphtalimid, Pd(acac)₂ 0,08 mol% (6,3 mg), 28,4 mg BuPoX, 10 mL Methylformiat, 10mL Methanol und 20µL Methansulfonsäure unter Schutzgas (z. B. Argon oder Stickstoff) in einen 100 mL Edelstahlautoklav überführt. Der Autoklav wird auf 100 °C erwärmt und bei dieser Temperatur 20 h gerührt. Anschließend wird der Autoklav abgekühlt auf Raumtemperatur und der Restdruck abgelassen. Es werden 5 mL Isooctan zur Reaktionslösung als interner Standard zugesetzt und das Gemisch wird gaschromatografisch untersucht. Die Ausbeute an n-Produkt, dem N-Phthaloyl-β-alanine methylester beträgt 56 %. Verzweigte Produkte waren nicht festzustellen. Somit ergibt sich eine Gesamtausbeute an Methylestern von 56 % mit einem n: iso Verhältnis von 100 : 0.

Aus den Beispielen sind die sehr guten Ausbeuten und Selektivitäten für das erfindungsgemäße Verfahren deutlich erkennbar.

## Patentansprüche

1. Verfahren zur Herstellung von Estern durch Carbonylierung, **dadurch gekennzeichnet dass**, es
i) unter Verwendung mindestens einer palladium-haltigen Verbindung,
ii) mindestens einer olefinisch ungesättigten Verbindung,
iii) mindestens eines phosphor-haltigen Liganden,
iv) mindestens eines Formiates,
v) mindestens eines Alkohols,
vi) mindestens einer Säure,
vii) in einem Temperaturbereich von 80° bis 120°C,
viii) bei einem Reaktionsdruck von 0,1 bis 0,6 MPa, durchgeführt wird,
wobei die Säure ausgewählt ist aus der Gruppe der Sulfonsäuren.

2. Verfahren nach Anspruch 1 **dadurch gekennzeichnet, dass** der Phosphor-haltige Ligand dreiwertigen Phosphor aufweist.

3. Verfahren nach Anspruch 2 **dadurch gekennzeichnet, dass** der Phosphor-haltige Ligand eine bidentate Struktur aufweist.

4. Verfahren nach Anspruch 3 **dadurch gekennzeichnet, dass** der Phosphor-haltige Ligand mit bidentater Struktur der Formel 1 entspricht.

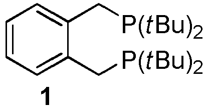

5. Verfahren nach Anspruch 1 **dadurch gekennzeichnet, dass** das Formiat ausgewählt ist unter Methylformiat, Ethylformiat, Benzylformiat, Phenylformiat.

6. Verfahren nach Anspruch 5 **dadurch gekennzeichnet, dass** der entsprechende Alkohol mit dem jeweils verwendeten Formiat korrespondiert.

7. Verfahren nach Anspruch 1 **dadurch gekennzeichnet, dass** die Sulfonsäuren ausgewählt sind aus Methansulfonsäure, p-Toluolsulfonsäure.

8. Verfahren nach Anspruch 1 **dadurch gekennzeichnet, dass** als olefinsich ungesättigte Verbindung olefinhaltige Gemische verwendet werden.

9. Verfahren nach Anspruch 8, **dadurch gekennzeichnet, dass** die Carbonylierung unter Isomerisierung zum n-terminalen Ester erfolgt.

10. Verfahren nach Anspruch 1 **dadurch gekennzeichnet, dass** die olefinisch ungesättigten Verbindungen Stickstoff und/oder Sauerstoff aufweisen.

11. Verfahren nach Anspruch 1 **dadurch gekennzeichnet, dass** die Palladium-haltige Verbindung mit Palladium in den Oxidationsstufen 0 bis +II ausgewählt ist aus der Gruppe enthaltend:
Pd-Halogenide,
Pd-Acetylacetonat,
Pd-Acetat,
Pd-Dibenzylidenaceton,
Pd-Triphenylphophin.
Abbildung 1: 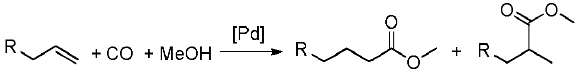
Abbildung 2:

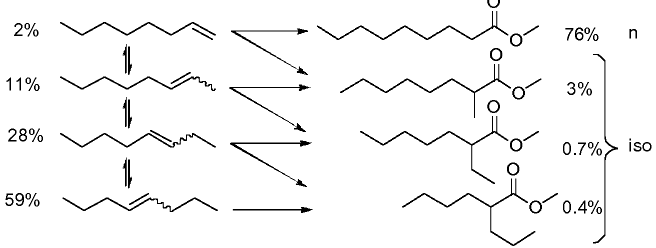

## Claims

1. Method of producing esters by carbonylation, **characterized in that** it is carried out
i) using at least one palladium-containing compound,
ii) at least one olefinically unsaturated compound,
iii) at least one phosphorus-containing ligand,
iv) at least one formate,
v) at least one alcohol,
vi) at least one acid,
vii) in a temperature range of 80°C to 120°C,
viii) at a reaction pressure of 0.1 to 0.6 MPa,
wherein the acid is selected from the group of sulphonic acids.

2. Method according to Claim 1, **characterized in that** the phosphorus-containing ligand comprises trivalent phosphorus.

3. Method according to Claim 2, **characterized in that** the phosphorus-containing ligand comprises a bidentate structure.

4. Method according to Claim 3, **characterized in that** the phosphorus-containing ligand of bidentate structure conforms to formula **1**:

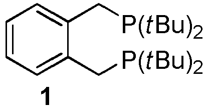

5. Method according to Claim 1, **characterized in that** the formate is selected from methyl formate, ethyl formate, benzyl formate, phenyl formate.

6. Method according to Claim 5, **characterized in that** the at least one alcohol corresponds to the particular formate used.

7. Method according to Claim 1, **characterized in that** the sulphonic acids are selected from methanesulphonic acid, p-toluenesulphonic acid.

8. Method according to Claim 1, **characterized in that** olefin-containing mixtures are used as olefinically unsaturated compounds.

9. Method according to Claim 8, **characterized in that** the carbonylation is effected with isomerization to the n-terminal ester.

10. Method according to Claim 1, **characterized in that** the olefinically unsaturated compounds comprise nitrogen and/or oxygen.

11. Method according to Claim 1, **characterized in that** the palladium-containing compound with palladium in the oxidation states 0 to +II is selected from the group containing:
palladium halides,
palladium acetylacetonate,
palladium acetate,
palladium dibenzylideneacetone,
palladium triphenylphosphine.

## Revendications

1. Méthode de production d'esters par carbonylation, **caractérisée en ce qu'**elle est effectuée
i) en utilisant au moins un composé à base de palladium,
ii) au moins un composé oléfiniquement insaturé,
iii) au moins un ligand phosphoré,
iv) au moins un formiate,
v) au moins un alcool,
vi) au moins un acide,
vii) dans une plage de température allant de 80°C à 120°C,
viii) à une pression de réaction allant de 0,1 à 0,6 MPa,
où l'acide est choisi dans le groupe constitué par les acides sulfoniques.

2. Méthode selon la revendication 1, **caractérisée en ce que** le ligand phosphoré comprend du phosphore trivalent.

3. Méthode selon la revendication 2, **caractérisée en ce que** le ligand phosphoré comprend une structure bidentée.

4. Méthode selon la revendication 3, **caractérisée en ce que** le ligand phosphoré de structure bidentée répond à la formule **1** :

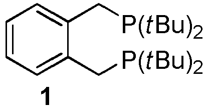

5. Méthode selon la revendication 1, **caractérisée en ce que** le formiate est choisi parmi le formiate de méthyle, le formiate d'éthyle, le formiate de benzyle, le formiate de phényle.

6. Méthode selon la revendication 5, **caractérisée en ce que** le au moins un alcool correspond au formiate particulier utilisé.

7. Méthode selon la revendication 1, **caractérisée en ce que** les acides sulfoniques sont choisis parmi l'acide méthanesulfonique, l'acide p-toluènesulfonique.

8. Méthode selon la revendication 1, **caractérisée en ce que** des mélanges à base d'oléfine sont utilisés comme composés oléfiniquement insaturés.

9. Méthode selon la revendication 8, **caractérisée en ce que** la carbonylation est effectuée par isomérisation à l'ester n-terminal.

10. Méthode selon la revendication 1, **caractérisée en ce que** les composés oléfiniquement insaturés comprennent de l'azote et/ou de l'oxygène.

11. Méthode selon la revendication 1, **caractérisée en ce que** le composé à base de palladium ayant du palladium dans les états d'oxydation 0 à +II est choisi dans le groupe constitué par :
les halogénures de palladium,
l'acétylacétonate de palladium,
l'acétate de palladium,
la dibenzylidèneacétone de palladium,
la triphénylphosphine de palladium.
